# EUROPEAN PATENT SPECIFICATION

(11) **EP 0 854 364 B1**
(45) Date of publication and mention of the grant of the patent: **28.08.2002**
(21) Application number: 98300342.7
(22) Date of filing: 19.01.1998
(51) Int. Cl.: G01N 33/68, G01N 33/541, G01N 33/535, G01N 23/04, C12Q 1/68, C07K 1/14

(54) **Diagnosis of neuro-degenerative disorders**
Diagnose von neuro-degenerativen Erkrankungen
Diagnostique de troubles neurodégénérescents

(30) Priority: 18.01.1997 GB 9701045
(43) Date of publication of application: 22.07.1998
(73) Proprietor: Biotec Global Limited, Newcastle Upon Tyne, NE1 8AS (GB)
(72) Inventor: Narang, Harash Kumar, Newcastle Upon Tyne, NE2 3DH (GB)
(74) Representative: Browne, Robin Forsythe, Dr.

(56) References cited:
- WO-A-90/15331
- WO-A-93/03369
- WO-A-96/17249
- WO-A-96/17250
- GB-A- 2 258 867
- CHEMICAL ABSTRACTS, vol. 122, no. 7, 13 February 1995 Columbus, Ohio, US; abstract no. 78275a, NARANG, H.K.: "Evidence that homologous ssDNA is present in scrapie, Creutzfeldt-Jakob disease, and bovin spongiform encephalopathy" page 780; XP002900053 & "Slow infections of the central nervous system" ANN. N.Y. ACAD. SCI., 1994, pages 314-326,
- DATABASE WPI Section Ch, Week 9111 Derwent Publications Ltd., London, GB; Class B04, AN 91-078708 XP002900054 & JP 03 027 349 A (ASAHI OPTICAL CO LTD)

## Description

The present invention relates to diagnosis of neuro-degenerative disorders in humans and animals.

Neuro-degenerative disorders include non-transmissible diseases such as Alzheimer's disease and multiple sclerosis, and spongiform encephalopathies.

Spongiform encephalopathies, such as Creutzfeldt-Jakob disease (CJD), Gerstmann-Straussler-Scheinker Syndrome (GSS) and Kuru in humans; scrapie in sheep and goats and bovine spongiform encephalopathy (BSE) in cattle, mink and cats are all transmissible (infective) neuro-degenerative disorders implicating vacuolation of neurons.

At present, the most reliable method of detecting an encephalopathy is histologically, especially by electron microscopy, but this requires brain tissue removed following autopsy of the dead victim. Although neurological examination and electro-encephalographs (EEG) can provide accurate diagnosis in many cases of encephalopathy, there is an urgent need for a definitive test during life, one which can detect the disease during its early stages and which is non-intrusive.

It is therefore the aim of the present invention to provide a means for the rapid and early diagnosis of encephalopathy using non-intrusive means such as a urine test.

The protein associated with for example the neuro-degenerative disorder CJD is thought to be a particle termed a "nemavirus". In contrast to the morphology of a common virus, which has a two layer structure of nucleic acid protected by an outer coat, the nemavirus particle has an unusual three layer structure which comprises:
1. a protein core,
2. single stranded DNA, and
3. a protein coat

The single stranded DNA is sandwiched between the protein core and the protein coat. Single stranded DNA from scrapie has been partly sequenced and contains a palindromic repeat sequence TACGTA. The scrapie-specific nucleic acid is single stranded DNA and includes the sequence (TACGTA)ₙ where n is at least 6. The basic six unit of this repeat sequence is palindromic, in the sense that a complementary DNA would have the same TACGTA sequence when read in the 5' to the 3' direction. The full length sequence of the DNA is not known, but it is suspected that n is very much larger than 6, perhaps of the order of 20 to 30. Although the DNA sequence is scrapie-specific, BSE, scrapie, CJD and other encephalopathies are thought to result from the same protein associated with the neuro-degenerative disorder transferred to another species. It is therefore believed that the TACGTA palindromic sequence appears in all known spongiform encephalopathies and possibly others.

The protein coat has not yet been characterised. The protein core comprises the protease-resistant protein (PrP) which is termed a "prion". A prion is encoded by a cellular gene of the host and is thought to contain little or no nucleic acid. However, the cellular form of the prion protein is modified into protease-resistant protein (PrP), by an accessory protein, "Nemo Corrupta" coded by single stranded DNA (PESM, 212, 208-224, (1996). This feature distinguishes prions sharply from virions. To date, no prion-specific nucleic acid which is required for transmission of disease has been identified.

Virus-like nemaviruses are tubulofilamentous particles in shape, typically 23-26 nm in diameter. They are consistently detected in the brains of all known spongiform encephalopathies. These particles have a core of prion in a rod-like form; the prion rods being also termed scrapie-associated fibrils (SAF). Over the core is a layer of DNA, removable by DNAse; above the core is an outer protein coat which is digestible by a protease.

It would be desirable to have a method of diagnosis based on nucleic acid identification or on the core structure of the nemavirus protease-resistant protein in a living human or animal. Such methods have been suggested where a probe of DNA derived from the gene sequence coding for a prion protein are used. However, since it is well known that prion protein is encoded by a normal chromosomal gene found in all mammals, including those affected by encephalopathies, the above work has not gained acceptance. PCT Patent Application WO89/11545 (Institute for Animal Health Ltd) purports to describe a method of detection of scrapie susceptibility by use of a restriction fragment length polymorphism (RFLP) linked to the so called Sinc gene associated with short incubation times of sheep infected by scrapie. The RFLP is said to be located in a non-coding portion associated with the gene for the prion. At best, this method would detect only sheep with the short incubation time characteristic. Hitherto, methods of diagnosis based on nucleic acid identification have not been very successful or are likely to be unsuccessful, since an encephalopathy specific nucleic acid has eluded detection despite numerous attempts.

In human CJD cases, the protein associated with the neuro-degenerative disorder has been consistently shown by titration studies to be present in blood. Although the protein associated with the neuro-degenerative disorder is present in urine of CJD cases, there is no known technique of diagnosis based on urine.

I have now developed a diagnostic method, which may be quantitative or semi-quantitative, in which some estimate is made of the amount of the protein associated with the neuro-degenerative disorder from a urine specimen which can be collected from the live animal.

I have previously described, in patent number GB 2258867, a method for the diagnosis of encephalopathy using animal tissue. This method includes the use of a scrapie-specific nucleic acid, part of which can be labelled and used as an oligonucleotide probe in a hybridisation assay. Alternatively, a sequence from the scrapie-specific nucleic acid is used as a primer in a polymerase chain reaction to make sufficient quantities to allow detection by a restriction fragment length method.

WO90/15331 discloses a diagnostic Alzheimer's disease assay in which protein material in a body fluid is concentrated using ultrafiltration.

The method according to the invention of diagnosing at least one neuro-degenerative disorder in an animal (such as a human), which neuro-degenerative disorder has specific protein material associated therewith, which method comprises:
(a) providing a sample of body fluid obtained from said animal;
(b) contacting said sample with solid, non-buoyant granular calcium phosphate having free ionic valencies, such that when said protein material associated with said neuro-degenerative disorder is present in said sample, said protein material in said sample is aggregated on the surface of said calcium phosphate; and
(c) monitoring said protein material which has aggregated on said surface.

Calcium phosphate is widely used in transformation experiments to allow the introduction of DNA into a living cell, wherein it causes the precipitation of DNA. However, it has not been previously suggested for the purpose of concentrating a protein material associated with neuro-degenerative disorder in a diagnostic sample of urine or the like.

Part of the protein associated with the neuro-degenerative disorder (in the case of spongiform encephalopathies) or amyloid precursor protein APP (in the case of a non-transmissible neuro-degenerative disease, such as Alzheimer's and basic myelin protein oligocyte for multiple sclerosis) binds or aggregates to the surface of the granules of calcium phosphate.

The steps leading to the concentration of a protein material associated with an infectious neuro-degenerative disorder from a sample of urine typically comprise:
(a) collecting and centrifuging a sample of urine from an animal suspected of being infected with said disorder;
(b) collecting the supernatant produced following centrifugation of said sample of urine;
(c) adding a buffer and solid, non-buoyant granular calcium phosphate having free ionic valencies to said supernatant;
(d) centrifuging the resulting mixture of said buffer, said calcium phosphate and said supernatant;
(e) collecting said calcium phosphate following centrifugation;
(f) adding a buffer to said calcium phosphate;
(g) centrifuging said mixture of said buffer and said calcium phosphate;
(h) collecting said calcium phosphate;
(i) adding a buffer to said calcium phosphate;
(j) centrifuging a mixture of said buffer and said calcium phosphate; and
(k) collecting said calcium phosphate having said protein material aggregated thereon, such that said protein material is in a concentration suitable for monitoring said protein material.

The concentration of the protein material in the sample of urine or the like can be increased 100 fold or more using calcium phosphate in the method according to the invention; the aggregated protein material can then be used in several ways to allow diagnosis of a neuro-degenerative disorder.

According to one aspect of the present invention, the aggregated protein material can be used for the detection of tubulofilamentous particles using electron microscopy. In such a method, a grid is brought into contact with the protein material and then the grid is fixed and stained. This allows the tubulofilamentous particles that are characteristic of the nemavirus or protein associated with the neuro-degenerative disorder to be visualized by electron microscopy.

Diagnosis of encephalopathy can alternatively be carried out by means of, for example, an enzyme-linked immunosorbent assay (ELISA). The ELISA technique can be automated to provide a semi-quantitative result. The calcium phosphate for the concentration of the nemavirus would be included as part of an ELISA kit. Such a kit according to the invention preferably further comprises a blocking buffer, an antibody to PrP and an antibody conjugate. A kit according to the invention preferably comprises:
(a) solid, non-buoyant granular calcium phosphate having free ionic valencies in a form capable of aggregating protein material associated with a neuro-degenerative disorder present in a sample of body fluid;
(b) a blocking buffer capable of complexing with residual said calcium phosphate not aggregated with said protein material;
(c) a first antibody material capable of complexing with said aggregated protein material; and
(d) a second antibody which is capable of complexing with said first antibody.

When the neuro-degenerative disorder is a spongiform encephalopathy, an antibody to PrP may be added which will bind to the protein material aggregated on the surface of the calcium phosphate. This is generally followed by a second antibody which will bind to the previous antibody, the second antibody being conjugated to a marker enzyme to allow detection of the protein material associated with the neuro-degenerative disorder.

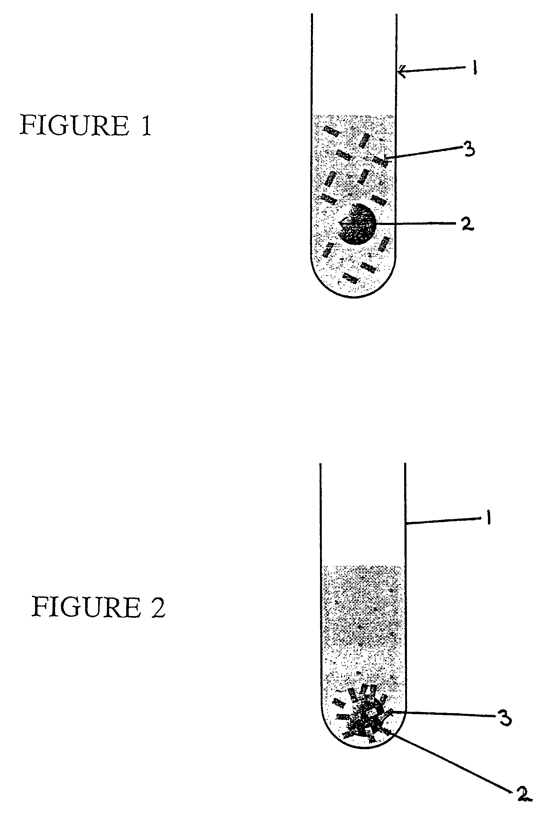

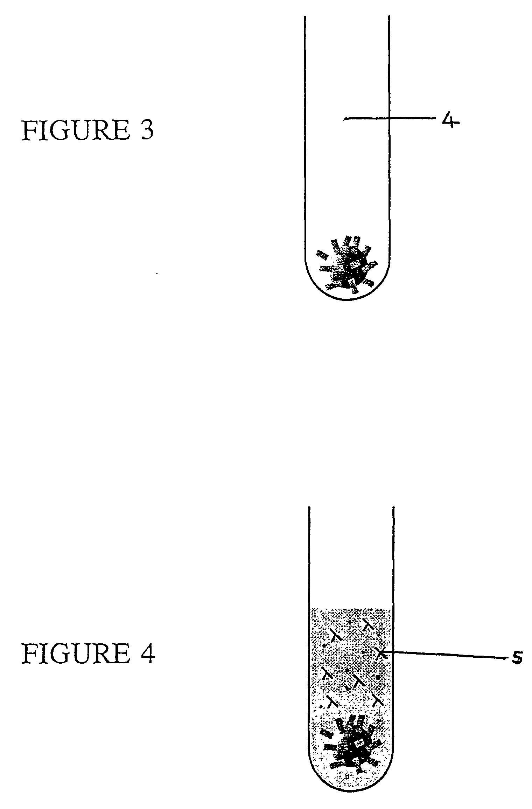

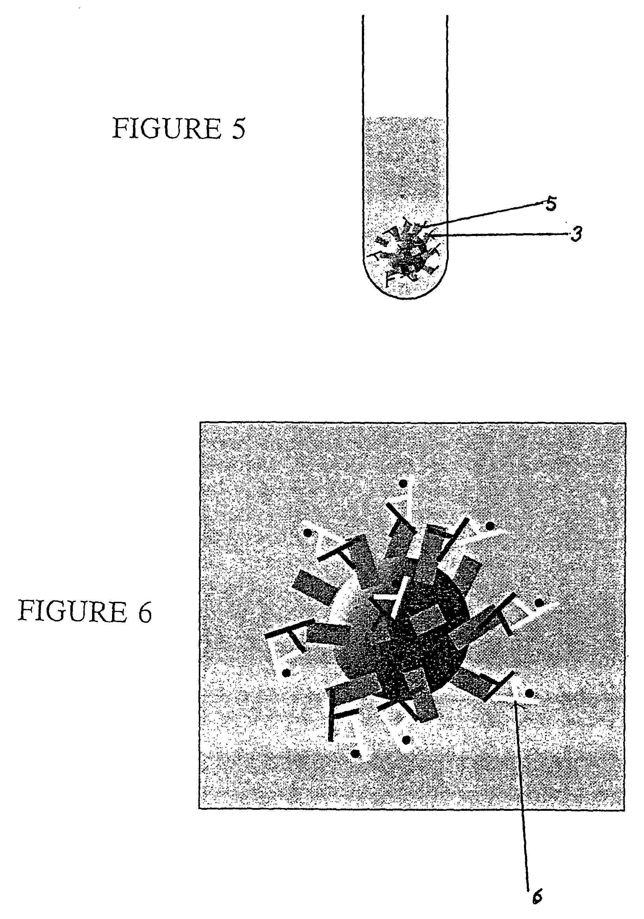

The use of calcium phosphate in the concentration of the protein associated with the neuro-degenerative disorder and the subsequent detection using an ELISA method is shown schematically in Figures 1 to 6 of the accompanying drawings, which are by way of example only, and in which:
Figure 1 shows a reaction vessel 1, having therein an exemplary calcium phosphate granule 2 and a protein 3 associated with a neuro-degenerative disorder;
Figure 2 shows the protein 3 associated with the neuro-degenerative disorder concentrated on the surface of the calcium phosphate granule 2;
Figure 3 shows the unbonded sites on the surface of the calcium phosphate granule 2 blocked on the addition of blocking buffer (such as milk) 4;
Figure 4 shows the addition of a first antibody against the protein associated with a neuro-degenerative disorder 5;
Figure 5 shows binding of the first antibody 5 to the protein 3 associated with a neuro-degenerative disorder which is still bonded to the surface of the calcium phosphate granule 2;
Figure 6 shows antibody detection using a second antibody 6 conjugated to a markers enzyme such as horseradish peroxidase or alkaline phosphatase; and
Figure 7 is a photograph of a stained blot obtained in an exemplary diagnostic method according to the invention.

Another method for the diagnosis of encephalopathy from the material aggregated on the surface of the calcium phosphate according to the invention is to amplify the DNA in the aggregated material by using a polymerase chain reaction (PCR). In a preferred method, the palindromic oligonucleotide described above is used to amplify the DNA. Such oligonucleotides will not normally be longer than 200 nucleotides, even when used as probes; generally, they are likely to be very much shorter. Thus, for PCR purposes they are unlikely to comprise more than 24 nucleotides of the palindrome, plus an optional 5'-end or tail of (say) 8 to 20 nucleotides, making 32 to 44 nucleotides in all. The PCR will yield a product in the form of DNA of varying lengths containing the palindromic sequence. This can preferably be analysed by a method relying on restriction by an enzyme.

The PCR product will produce bands of various molecular weights. In some instances the encephalopathy-specific DNA will be primed near its 3'-end, which will generate multiple copies of large molecules. The PCR product may be divided into two portions, of which the first may be run on a resolving gel to show a band of high molecular weight associated with the encephalopathy-specific DNA, the second portion being restricted with a restriction enzyme which cuts the palindromic sequence. This restriction will severely reduce the length of the longer DNA and eliminate certain other bands of shorter DNA altogether. Multiple restrictions of TACGTA will produce many bands of molecular weight too low to be detected. Restricted product can be compared with the unrestricted product, whereby disappearance of longer lengths of DNA upon restriction indicates the presence of the encephalopathy-specific DNA in the aggregated material.

Examples of suitable restriction enzymes are SnaBI and AccI, which cut between the C and G of TACGTA and Bst11071 which cuts between A and T of one TACGTA sequence and the next TACGTA sequence. Such enzymes recognise the six-base sequence and leave blunt ends.

The aggregated protein material associated with the neuro-degenerative disorder can be used in a further assay for the diagnosis of encephalopathy, using a hybridisation method. In the hybridisation method, DNA in the aggregated material can be used as it is, or preferably, it may be amplified before use, for example, using a PCR method. The hybridisation probe is preferably from 16 to 100 nucleotides long, especially about 40 nucleotides long. The hybridisation assay can be carried out in a conventional manner; Southern blotting is preferred. For use in a hybridisation assay, the oligonucleotide will normally be used in a labelled form, labelling being by any appropriate method such as radiolabelling, for example, by ³²P or ³⁵S, or by biotinylation (which can be followed by reaction with labelled avidin). However, it is also possible to use an unlabelled oligonucleotide as a probe provided that it is subsequently linked to a label. For example, the oligonucleotide could be provided with a poly-C tail which could be linked subsequently to labelled poly-G.

An alternative method for the diagnosis of encephalopathy is using a protein blotting method (Western blotting) which comprises detecting the protein of interest on the surface of a membrane (such as nitrocellulose) using antibody technology.

### Methods and Materials

### 1. Purification of a Protein material associated with a NeuroDegenerative Disorder from a sample (for example urine)

A sample of urine was collected from an animal suspected of being infected. The urine sample was centrifuged and the supernatant collected. A suitable buffer and calcium phosphate granules were then added to the supernatant. This mixture of urine supernatant, buffer and calcium phosphate was allowed to rest at room temperature (with regular mixing) for at least ten minutes. The mixture was then centrifuged and the calcium phosphate granules collected. A suitable buffer was then added to the calcium phosphate granules followed by a further centrifugation step. The calcium phosphate granules were collected and the above addition of buffer and centrifugation step was repeated a further two times. The calcium phosphate granules were collected for the detection of a possible protein associated with a neurodegenerative disorder using any of methods 2,3,4,5,6 and 7 as follows.

### 2. Enzyme Linked Immunosorbent Assay

The calcium phosphate granules obtained following the above purification stage were used.
A suitable blocking buffer (for example milk) was added to the calcium phosphate granules and the solution was left mixing for at least sixty minutes. The solution was then centrifuged and the supernatant discarded. To the calcium phosphate granules that remain some phosphate buffered saline (PBS) containing Tween 20 was added and this was followed by a further centrifugation step. The above PBS-Tween 20 wash step was repeated at least four times. A first antibody was then added to the calcium phosphate granules. This was left to stand for at least 60 minutes with mixing at regular intervals. PBS-Tween 20 was added and followed by a centrifugation step. The supernatant was discarded and the PBS-Tween 20 wash step repeated at least four times. A second antibody which will bind to the first antibody and is conjugated to a marker enzyme was then added to the calcium phosphate granules and left mixing for at least sixty minutes. PBS-Tween 20 was then added followed by a centrifugation step. The supernatant was discarded and the wash step repeated with PBS-Tween 20 at least four times. A substrate suitable for detection of the marker enzyme on the second antibody was then added. This was left to stand for at least twenty minutes and the reaction stopped by addition of a suitable reagent, such as concentrated sulphuric acid. Following centrifugation, the supernatant was collected and the product read photometrically at a suitable wavelength.

### 3. Preparation of grids for electron microscopy.

The calcium phosphate granules obtained following the purification steps outlined in method 1 were used.
Ethylenediaminetetraacetic acid (EDTA) was added to the calcium phosphate granules and mixed until a clear solution was produced. A carbon-coated grid was lowered into tubes containing some distilled water making sure the carbon/Formvar film was facing upwards. For each specimen at least two grids were prepared using the clear solution which was then transferred into the tube whilst gently rinsing off the distilled water. The grids were then centrifuged horizontally. After the centrifugation step sodium dodecyl sulphate was added and the grids transferred into distilled water. The grids were then washed several times in distilled water. The water was then momentarily replaced with glutaraldehyde containing ruthenium red. This solution was then rinsed out with distilled water and the grids were then momentarily introduced to a solution of osmic acid containing ruthenium red. The grids were again rinsed several times with distilled water. After the final wash of water with a drop of phosphotungstic acid the grids were dried and examined under an electron microscope.

### 4. Polymerase Chain Reaction

Again the calcium phosphate granules obtained following the purification stage (method 1) were used.
EDTA was added to the calcium phosphate granules until a clear solution was produced. Some of this clear solution was taken and incubated with proteinase K for at least one hour at 55°C. The proteinase K was then heat inactivated at 95°C and the solution used as a template in a polymerase chain reaction (PCR). A dNTP mix, primers, a buffer and AmpliTaq DNA polymerase were then added to the reaction mixture. Thirty cycles of PCR were carried out comprising a denaturation stage, annealing of primers and an extension stage. The PCR product was then cut with the restriction enzyme SnaB1. Cut and uncut PCR product was then analysed using electrophoresis and the fragments visualised on the agarose gel after staining with ethidium bromide.

### 5. Protein Blotting for immunoassay

Bio-Dot apparatus was used for the immunoblotting procedure. Nitrocellulose membranes were pre-wetted in Tris saline buffer (TSB) prior to placing in the bio-dot apparatus. After rehydrating the membrane the wells of the apparatus were filled with antigen. The antigen solution being the clear solution produced on mixing the calcium phosphate granules from method 1 with EDTA). The entire antigen sample was allowed to filter through the membrane. After the antigen samples had completely drained from the apparatus the Tris saline buffer (TSB) was added and the liquid allowed to filter through. Blocking solution was then added to each well and the liquid allowed to filter through the apparatus. Tween-tris saline buffer (TTSB) wash solution was added to the apparatus and the flow valve adjusted to produce a vacuum to pull the wash solution through the membrane. The vacuum was then disconnected and a first antibody solution added to each sample well. The solution was allowed to filter through the membrane and the vacuum was re-applied to remove any excess liquid from the sample wells. TTSB wash solution was then added and pulled through the membrane with the aid of a vacuum. The wash process was then repeated three times.

Conjugated antibody solution was added to each well and the liquid allowed to filter through. TTSB wash solution was then added to each well and the solution pulled through the membrane with the aid of a vacuum. This wash process was repeated twice. The membrane was removed and placed in the colour development vessel. The membrane was then removed and washed with TSB to remove excess Tween 20. The membrane was then incubated in a suitable substrate until the development of spots were seen. After this time the membrane was rinsed in distilled water and photographed.

### 6. Southern Blotting

A solution obtained from the calcium phosphate granules (method 1) was taken and concentrated NaOH and DMSO added. The solution was mixed and heated and then cooled down to room temperature after which concentrated ammonium acetate was added. Nitrocellulose membrane was then wetted in 6XSSC and the bio-dot apparatus assembled. The solution was applied to the membrane and allowed to filter through the membrane. After the sample had filtered 2XSSC was added to each well and vacuum applied to remove the liquid. The blot membrane was removed and washed in 2XSSC. The nitrocellulose membrane was then baked under vacuum before hydridisation.

### 7. Western Blotting

The calcium phosphate granules obtained following the purification steps outlined in method 1 were used. Sodium dodecyl sulphate containing proteinase k was then added to the calcium phosphate granules and the mixture incubated for at least 60 minutes at 55°C. β-mercaptoethanol was added after the incubation period and the mixture was then boiled. Following this polyacrylamide gel electrophoresis was carried out. Proteins on the polyacrylamide gel were then transferred to a nitrocellulose membrane. The membrane was air dried and then washed in tris buffered saline. Any unabsorbed sites were then blocked using heat inactivated horse serum and goat milk. A first antibody made up in tris-buffered saline containing Tween 20 and milk was then applied to the membrane which was left to incubate for at least one hour. The membrane was then washed several times. A second antibody conjugated to a marker enzyme (which was also made up in a solution of tris-buffered saline containing Tween 20) was then applied to the membrane. This was left to incubate for at least 60 minutes and then washed in a solution of tris buffered saline to remove excess Tween 20. The membrane was then incubated in a suitable substrate until the development of bands were seen. After this time the membrane was rinsed in distilled water and photographed.

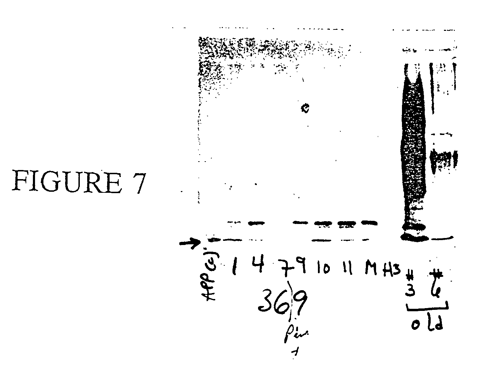

In an exemplary method, beta-amyloid protein (APP) was concentrated from urine specimens of patient having Alzheiemer's by the method described above and a Western blot performed. The resulting blot, stained by APP-antibody 369, is shown in Figure 7 of the accompanying drawings. Positive results are seen in lane 0, control APP, lanes 1,3,4,6,9,10,11 and M from specimens from Alzheimer's patients.

Lane 3 is control and lane 7 relates to an assay for specimens from patients with Parkinson's disease.

## Claims

1. A method of diagnosing at least one neuro-degenerative disorder in an animal (such as a human), which neuro-degenerative disorder has specific protein material associated therewith, which method comprises:
(a) providing a sample of body fluid obtained from said animal;
(b) contaction said sample with solid, non-buoyant granular calcium phosphate having free ionic valencies, such that when said protein material associated with said neuro-degenerative disorder is present in said sample, said protein material in said sample is aggregated on the surface of said calcium phosphate; and
(c) monitoring said protein material which has aggregated on said surface.

2. A method according to claim 1, wherein said aggregated protein material is monitored in step (c) using electron microscopy.

3. A method according to claim 1, wherein said aggregated protein material is monitored in step (c) using an enzyme linked immunosorbent assay (ELISA) or Western blotting or dot blot.

4. A method according to claim 3, in which a first antibody is added to said aggregated protein material so as to permit said first antibody to complex with said aggregated protein material.

5. A method according to claim 4, wherein a second antibody which is conjugated to a marker enzyme is added to said aggregated protein material complexed with said first antibody so as to permit said second antibody to complex to said first antibody.

6. A method according to claim 1, wherein step (c) comprises amplifying DNA associated with said aggregated protein material using a polymerase chain reaction and then analysing said amplified DNA material by a restriction fragment length method.

7. A method according to claim 6, wherein step (c) further comprises using said amplified DNA material in a hybridisation reaction such as Southern blotting.

8. A method according to claim 1, wherein step (c) comprises using DNA associated with said aggregated protein material in a hybridisation reaction such as Southern blotting.

9. A method according to any preceding claim, wherein a buffer is added to said sample and said calcium phosphate in step (b).

10. A kit for carrying out an ELISA reaction, the kit comprising:
(a) solid, non-buoyant granular calcium phosphate having free ionic valencies in a form capable of aggregating protein material associated with a neuro-degenerative disorder present in a sample of body fluid;
(b) a blocking buffer capable of complexing with residual calcium phosphate not aggregated with said protein material;
(c) a first antibody material capable of complexing with said aggregated protein material; and
(d) a second antibody which is capable of complexing with said first antibody.

11. A method for concentrating a protein material associated with an infectious neuro-degenerative disorder from a sample of urine, which comprises the following steps:
(a) collecting and centrifuging a sample of urine from an animal suspected of being infected with said disorder;
(b) collecting the supernatant produced following centrifugation of said sample of urine;
(c) adding a buffer and solid, non-buoyant granular calcium phosphate having free ionic valencies to said supernatant;
(d) centrifuging the resulting mixture of said buffer, said calcium phosphate and said supernatant, such that when said protein material associated with said neuro-degenerative disorder is present in said sample of urine, said protein material is aggregated on the surface of said calcium phosphate;
(e) collecting said calcium phosphate following centrifugation;
(f) adding a buffer to said calcium phosphate;
(g) centrifuging said mixture of said buffer and said calcium phosphate;
(h) collecting said calcium phosphate;
(i) adding a buffer to said calcium phosphate;
(j) centrifuging a mixture of said buffer and said calcium phosphate; and
(k) collecting said calcium phosphate having said protein material aggregated thereon, such that said protein material is in a concentration suitable for monitoring said protein material.

## Patentansprüche

1. Verfahren zur Diagnose wenigstens einer neuro-degenerativen Erkrankung in einem Tier (wie einem Menschen), wobei die neuro-degenerative Erkrankung mit einem spezifischen Proteinmaterial assoziiert ist und man bei dem Verfahren:
(a) eine Probe Körperflüssigkeit, die von dem Tier gewonnen wird, bereitstellt,
(b) die Probe mit festem, nicht-schwimmfähigen gekörnten Calciumphosphat mit freien Ionenvalenzen zusammenbringt, so dass, wenn das mit der neuro-degenerativen Erkrankung assoziierte Proteinmaterial in der Probe vorkommt, das Proteinmaterial aus der Probe auf der Oberfläche des Calciumphosphats aggregiert; und
(c) das Proteinmaterial, das auf der Oberfläche aggregiert, überwacht.

2. Verfahren nach Anspruch 1, wobei das aggregierte Proteinmaterial in Schritt (c) mittels Elektronenmikroskopie überwacht wird.

3. Verfahren nach Anspruch 1, wobei das aggregierte Proteinmaterial in Schritt (c) mit einem Festphasen-Enzymimmunoassay (ELISA) oder durch Western Blotting oder durch einen Dot-Blot überwacht wird.

4. Verfahren nach Anspruch 3, wobei man einen ersten Antikörper zu dem aggregierten Proteinmaterial zugibt, so dass der erste Antikörper mit dem aggregierten Proteinmaterial einen Komplex bilden kann.

5. Verfahren nach Anspruch 4, wobei man einen zweiten Antikörper, der an ein Markerenzym konjugiert ist, zu dem mit dem ersten Antikörper im Komplex gebundenen aggregierten Proteinmaterial gibt, so dass der zweite Antikörper mit dem ersten Antikörper einen Komplex bilden kann.

6. Verfahren nach Anspruch 1, wobei Schritt (c) das Amplifizieren von DNA umfasst, die mit dem aggregierten Proteinmaterial assoziiert ist, wobei man eine Polymerase-Kettenreaktion verwendet und das amplifizierte DNA-Material anschließend durch ein Verfahren zur Bestimmung der Restriktionsfragmentlängen untersucht.

7. Verfahren nach Anspruch 6, wobei man in Schritt (c) ferner das amplifizierte DNA-Material in einer Hybridisierungsreaktion, wie dem Southern Blotting, verwendet.

8. Verfahren nach Anspruch 1, wobei man in Schritt (c) mit dem aggregierten Proteinmaterial assoziierte DNA in einer Hybridisierungsreaktion, wie dem Southern Blotting, verwendet.

9. Verfahren nach einem der vorstehenden Ansprüche, wobei man zu der Probe und dem Calciumphosphat in Schritt (b) einen Puffer zugibt.

10. Kit zur Durchführung einer ELISA-Reaktion, umfassend:
(a) festes, nicht-schwimmfähiges gekörntes Calciumphosphat mit freien Ionenvalenzen in einer Form, in der es fähig ist Proteinmaterial zu aggregieren, das mit einer neuro-degenerativen Erkrankung assoziiert ist und in einer Probe Körperflüssigkeit vorkommt;
(b) einen Blockierungspuffer, der fähig ist mit restlichem Calciumphosphat zu aggregieren, das nicht mit dem Proteinmaterial aggregiert ist;
(c) ein erstes Antikörpermaterial, das fähig ist mit dem aggregierten Proteinmaterial einen Komplex zu bilden; und
(d) einen zweiten Antikörper, der fähig ist mit dem ersten Antikörper einen Komplex zu bilden.

11. Verfahren zum Aufkonzentrieren eines Proteinmaterials, das mit einer infektiösen neuro-degenerativen Erkrankung assoziiert ist, aus einer Harnprobe, umfassend die nachstehenden Schritte:
(a) Auffangen und Zentrifugieren einer Harnprobe von einem Tier, das unter dem Verdacht steht mit der Erkrankung infiziert zu sein;
(b) Auffangen des Überstands, der nach dem Zentrifugieren der Harnprobe entsteht;
(c) Zugeben eines Puffers und festes, nicht-schwimmfähiges gekörntes Calciumphosphat mit freien Ionenvalenzen zu dem Überstand;
(d) Zentrifugieren des erhaltenen Gemischs aus dem Puffer, dem Calciumphosphat und dem Überstand, so dass, wenn das mit der neuro-degenerativen Erkrankung assoziierte Proteinmaterial in der Harnprobe vorkommt, das Proteinmaterial auf der Oberfläche des Calciumphosphats aggregiert;
(e) Auffangen des Calciumphosphats nach der Zentrifugation;
(f) Zugeben eines Puffers zu dem Calciumphosphat;
(g) Zentrifugieren des Gemischs aus dem Puffer und dem Calciumphosphat;
(h) Auffangen des Calciumphosphats;
(i) Zugeben eines Puffers zu dem Calciumphosphat;
(j) Zentrifugieren eines Gemischs aus dem Puffer und dem Calciumphosphat, und
(k) Auffangen des Calciumphosphats mit dem darauf aggregierten Proteinmaterial, so dass das Proteinmaterial in einer für die Überwachung des Proteinmaterials geeigneten Konzentration vorliegt.

## Revendications

1. Procédé pour diagnostiquer au moins une affection neurodégénérative chez un animal (tel qu'un humain), laquelle affection neurodégénérative présente un matériel protéique spécifique qui lui est associé, lequel procédé comprend :
(a) la fourniture d'un échantillon de fluide corporel obtenu dudit animal ;
(b) la mise en contact dudit échantillon avec du phosphate de calcium solide, non-flottable, granuleux, ayant des valences ioniques libres, de telle sorte que quand ledit matériel protéique associé à ladite affection neurodégénérative est présent dans ledit échantillon, ledit matériel protéique dans ledit échantillon s'agrège à la surface dudit phosphate de calcium ; et
(c) le suivi dudit matériel protéique qui s'est agrégé sur ladite surface.

2. Procédé selon la revendication 1, dans lequel ledit matériel protéique agrégé est suivi dans l'étape (c) en utilisant la microscopie électronique.

3. Procédé selon la revendication 1, dans lequel ledit matériel protéique agrégé est suivi dans l'étape (c) en utilisant un essai d'immuno-absorption enzymatique (ELISA), un transfert de Western ou un transfert en point.

4. Procédé selon la revendication 3, dans lequel un premier anticorps est ajouté au dit matériel protéique agrégé pour permettre au dit premier anticorps de se complexer avec ledit matériel protéique agrégé.

5. Procédé selon la revendication 4, dans lequel un deuxième anticorps, qui est conjugué à une enzyme marqueur, est ajouté au dit matériel protéique agrégé complexé avec ledit premier anticorps pour permettre au dit deuxième anticorps de se complexer au dit premier anticorps.

6. Procédé selon la revendication 1, dans lequel l'étape (c) comprend l'amplification d'ADN associé au dit matériel protéique agrégé en utilisant une réaction en chaîne par polymérase et puis en analysant ledit ADN amplifié par une méthode d'analyse de la longueur des fragments de restriction.

7. Procédé selon la revendication 6, dans lequel l'étape (c) comprend en plus l'utilisation dudit ADN amplifié dans une réaction d'hybridation telle que le transfert de Southern.

8. Procédé selon la revendication 1, dans lequel l'étape (c) comprend l'utilisation d'un ADN associé au dit matériel protéique agrégé dans une réaction d'hybridation telle que le transfert de Southern.

9. Procédé selon l'une quelconque des revendications précédentes, dans lequel un tampon est ajouté au dit échantillon et au dit phosphate de calcium dans l'étape (b).

10. Kit pour exécuter une réaction ELISA, le kit comprenant ;
(a) du phosphate de calcium solide, non-flottable, granuleux ayant des valences ioniques libres sous une forme capable d'agréger un matériel protéique associé à une affection neurodégénérative présent dans un échantillon de fluide corporel ;
(b) un tampon de blocage capable de se complexer avec du phosphate de calcium résiduel non-agrégé avec ledit matériel protéique ;
(c) un premier anticorps capable de se complexer avec ledit matériel protéique agrégé ; et
(d) un deuxième anticorps qui est capable de se complexer avec ledit premier anticorps.

11. Procédé pour concentrer un matériel protéique associé à une affection neurodégénérative infectieuse d'un échantillon d'urine qui comprend les étapes suivantes :
(a) le prélèvement et la centrifugation d'un échantillon d'urine d'un animal suspecté d'être infecté par ladite affection ;
(b) le prélèvement du surnageant produit après centrifugation dudit échantillon d'urine ;
(c) l'ajout au dit surnageant d'un tampon et de phosphate de calcium solide, non-flottable, granuleux ayant des valences ioniques libres ;
(d) la centrifugation du mélange résultant desdits tampon, phosphate de calcium et surnageant de telle sorte que quand ledit matériel protéique associé à ladite affection neurodégénérative est présent dans ledit échantillon d'urine, ledit matériel protéique s'agrège à la surface dudit phosphate de calcium ;
(e) le prélèvement dudit phosphate de calcium après centrifugation ;
(f) l'ajout d'un tampon au dit phosphate de calcium ;
(g) la centrifugation dudit mélange dudit tampon et dudit phosphate de calcium ;
(h) le prélèvement dudit phosphate de calcium ;
(i) l'ajout d'un tampon au dit phosphate de calcium ;
(j) la centrifugation d'un mélange dudit tampon et dudit phosphate de calcium ; et
(k) le prélèvement dudit phosphate de calcium à la surface duquel s'est agrégé ledit matériel protéique de telle sorte que ledit matériel protéique soit à une concentration convenable pour le suivi dudit matériel protéique.
